# EUROPEAN PATENT SPECIFICATION

(11) **EP 0 441 245 B1**
(45) Date of publication and mention of the grant of the patent: **14.09.1994**
(21) Application number: 91101277.1
(22) Date of filing: 31.01.1991
(51) Int. Cl.: A61K 9/22

(54) **Method for the preparation of sustained-release medicated tablets**
Verfahren zur Herstellung arzneistoffhaltiger Tabletten mit verzögerter Freigabe
Procédé de préparation de comprimés médicamenteux à libération retardée

(30) Priority: 08.02.1990 JP 29237/90
(43) Date of publication of application: 14.08.1991
(73) Proprietor: SHIN-ETSU CHEMICAL CO., LTD., Chiyoda-ku Tokyo (JP)
(72) Inventor: Kokubo, Hiroyasu, Joetsu-shi, Niigata-ken (JP); Araume, Kiyoshi, Joetsu-shi, Niigata-ken (JP); Maruyama, Naosuke, Joetsu-shi, Niigata-ken (JP); Muto, Hiroaki, Joetsu-shi, Niigata-ken (JP)
(74) Representative: Blum, Rudolf Emil Ernst

(56) References cited:
- EP-A- 0 168 044
- BE-A- 688 121
- FR-A- 1 561 127
- GB-A- 2 163 648
- US-A- 3 147 187

## Description

### BACKGROUND OF THE INVENTION

The present invention relates to a method for the preparation of sustained-release medicated tablets capable of releasing the medicinally active ingredient at a constant rate in the patient's body administrated therewith. More particularly, the invention relates to a method for the preparation of sustained release medicated tablets of the matrix type.

Sustained-release medicated tablet is a useful medicament form capable of sustainedly releasing the medicinally active ingredient contained therein when it is administrated to a patient and has been studied and developed with an object to decrease the frequency of administration of the medicament to a patient by sustainedly maintaining the medicinal effect over a long time by a single administration and, when toxicity is exhibited or side effects are caused by the active ingredient with an excessively large concentration in blood to exceed a certain upper limit, to control the concentration thereof in blood not to exceed the critical value.

Several types of sustained-release medicated tablets are known in the prior art including the so-called matrix-type ones prepared by tableting a mixture of the active ingredient and a water-soluble polymeric material or a wax and the so-called Space-tab-type ones prepared by tableting a blend of rapid-release portions and sustained-release portions.

The active ingredient in the matrix-type sustained-release tablet is released therefrom by the motive force which is the concentration gradient of the active ingredient produced by the infiltration of water into the tablet so that it may be called a diffusion-controlled type. The gel matrix-type tablet by using a water-soluble polymeric material is prepared by tableting the active ingredient together with a base such as hydroxypropyl methyl cellulose, referred to as HPMC hereinbelow, either alone or as a combination with methyl cellulose, referred to as MC hereinbelow, sodium salt of carboxymethyl cellulose, referred to as Na-CMC hereinbelow, and the like. The gel-matrix type sustained-release medicated tablets are disclosed, for example, in Japanese Patent Kokai No. 58-174311. The wax-matrix type sustained-release medicated tablets are prepared from a powder of the active ingredient after a wax treatment according to the disclosure, for example, in Japanese Patent Kokai No. 62-10012.

These sustained-release tablets in the prior art have a problem in common relative to the amount of the HPMC or wax as a binding additive. When the amount of the additive is too large, namely, the releasing velocity of the active ingredient is unduly decreased in the later stage of releasing due to the decrease in the concentration gradient and increase in the distance of diffusion. When the amount of the additive is decreased in order to avoid the above mentioned phenomenon as far as possible, on the other hand, the tablet is sometimes disintegrated already in the early stage of releasing to release a large amount of the active ingredient at one time so that the concentration of the ingredient in blood can no longer be controlled at or below the desired upper limit or the tablet no longer serves as a sustained-release medicament form. When the active ingredient has high solubility in water, in particular, the amount of the binding additive must be increased so much that the size of the tablet would be too large for a medicinally active ingredient which must be administrated in a large dose.

The wax-matrix type tablet has another problem that, when it is kept under an adverse condition of high temperature, the releasing characteristic of the active ingredient from the tablet may be changed or, in some extreme cases, the tablet is excreted without disintegration. A disclosure is given in Japanese Patent Kokai No. 62-10012, so to say, on a combination of the gel-matrix type and wax-matrix type. The sustained-release tablet disclosed there is prepared in a process in which the active ingredient is dispersed in an aqueous solution of a water-soluble polymer such as HPMC and the like; the dispersion is dried and shaped into beads and the beads are treated with a molten wax and then shaped into tablets together with an excipient and lubricant. A problem in this prior art is the troublesomeness of the preparation process prohibiting the practical application of the process.

US-A-3 147 187 discloses a sustained release pharmaceutical consisting of a homogeneous mixture of the drug, fatty material (e.g. stearic acid, cetyl alcohol, beeswax) and swelling agent (e.g. methyl cellulose), prepared by heating the ingredients to melt.

GB-A-2 163 648 discloses a method of manufacturing a slowly releasing pharmaceutical, comprising mixing a gelling agent (e.g. methyl cellulose, hydroxypropyl cellulose, hydroxypropyl methyl cellulose), a fat/oil which is solid at room temperature (e.g. stearic acid, cetyl alcohol) and a pharmaceutical and then heating at the temperature which is not lower than the melting point of the fat/oil.

However, these documents of the state of the art do not disclose sustained release pharmaceutical compositions with good storage stability and a suppressed releasing velocity especially in the early stage.

### SUMMARY OF THE INVENTION

The present invention accordingly has an object to provide a novel method for the preparation of a sustained-release medicated tablet which is free from the problems of premature disintegration in the early stage of releasing in the patient's body and changes in the releasing characteristic by heating and capable of sustainedly controlling the concentration of the medicinally active ingredient in blood not to exceed the desired upper limit.

Thus, the method of the present invention for the preparation of a sustained-release medicated tablet comprises the steps of:
(a) blending a medicinally active ingredient with a non-ionic water-soluble cellulose ether and a wax to give a mixture, the amounts of the cellulose ether and the wax being in the ranges from 10 to 40% by weight and from 5 to 30% by weight, respectively, based on the total amount of the active ingredient, cellulose ether and wax;
(b) tableting the mixture into a tablet; and
(c) heating the tablet at a temperature lower than the melting point of the wax but not lower by more than 20 °C than said melting point for a length of time of at least 3 minutes and then cooling.

The sequential order of the steps (b) and (c) can be reversed.

### DETAILED DESCRIPTION OF THE PREFERRED EMBODIMENTS

As is described above, the most characteristic feature of the inventive method consists in the heat treatment of the tablet, which is a combination of the gel-matrix type and the wax-matrix type, under the very specific conditions. By virtue of this unique feature, the sustained-release tablet prepared according to the inventive method can be prevented from premature disintegration in the patient's body even with a relatively small amount of the binding additives.

One of the binding additives blended with the medicinally active ingredient in the inventive method is a non-ionic water-soluble cellulose ether which is selected from the group consisting of HPMC, MC and hydroxypropyl cellulose, referred to as HPC hereinbelow. These non-ionic water-soluble cellulose ethers can be used either singly or as a combination of two kinds or more according to need. Incidentally, ionic water-soluble cellulose ethers, such as Na-CMC, are not preferred in respect of the possible interaction with the active ingredient.

The above mentioned water-soluble cellulose ether forms a gel when it is contacted with water and the active ingredient contained in the tablet is released by diffusion through the thus formed gelled layer. Among the above mentioned three types of the non-ionic water-soluble cellulose ethers, HPMC and HPC are preferred in respect of the adequate gel-forming characteristics suitable for the inventive method. As to the average degree of polymerization of these cellulose ethers expressed by the viscosity of an aqueous solution thereof, it was found that a cellulose ether having a larger degree of polymerization could give a gelled layer having larger tenacity as compared with a cellulose ether of the same type but having a lower degree of polymerization so as to impart better controllability of the releasing velocity of the active ingredient in the tablet so that the releasing characteristic of the sustained-release tablet can be modified by adequately selecting the type of the cellulose ether and the average degree of polymerization thereof.

In the formulation of the tablet prepared according to the inventive method, the amount of the non-ionic water-soluble cellulose ether, which naturally depends on the desired releasing characteristic of the tablet, should be in the range from 10 to 40% by weight or, preferably, from 15 to 25% by weight based on the total amount of the active ingredient, cellulose ether and wax. When the amount of the cellulose ether is too small, the tablet prepared from the mixture of the components cannot be prevented from premature disintegration in the patient's body administrated therewith. When the amount thereof is too large, on the other hand, a disadvantage is caused that the releasing velocity of the active ingredient would be unduly decreased, in particular, in the later stage of releasing not to ensure constancy of the concentration of the active ingredient in blood of the patient.

The other of the binding additives blended with the medicinally active ingredient is a wax which should be insoluble or hardly soluble in water and have a melting point in the range from 50 to 90 °C or, preferably, from 55 to 70 °C. When the melting point of the wax is too low, the tablet prepared by using such a wax would be subject to a drawback of surface melting to cause a change in the releasing characteristic of the active ingredient when the tablet is kept at a high temperature. When the melting temperature of the wax is too high, on the other hand, the temperature of the heat treatment in step (c) of the inventive method must be so high that the active ingredient is possibly subject to thermal denaturation. It should be noted that, when the wax has no definite melting point but is melted in a certain range of temperature, the melting point of the wax here implied is contrued to be the lowest temperature at which melting of the wax begins when the temperature is gradually increased.

Various kinds of waxes can be used in the inventive method including various hardened oils such as hardened beef tallow melting at 60 °C, hardened rapeseed oil melting at 86 °C, hardened castor oil melting at 86 to 90 °C, paraffins such as paraffin waxes melting at 50 to 75 °C, microcrystalline wax melting at 70 to 90 °C, natural waxes such as carnauba wax melting at 78 to 84 °C, beeswax melting at 62 to 66 °C, bleached beeswax melting at 50 to 60 °C, higher alcohols such as cetyl alcohol melting at 50 °C, stearyl alcohol melting at 52 to 60 °C and higher fatty acids such as stearic acid melting at 70.1 °C. These waxy compounds can be used either singly or as a combination of two kinds or more according to need. Among the above named waxy compounds, hardened oils such as hardened beef tallow, hardened rapeseed oil and the like are particularly preferable in the inventive method in respect of the easiness in handling, high water resistance and strong hydrophobicity.

In the formulation of the tablet prepared according to the inventive method, the amount of the wax should be in the range from 5 to 30% by weight or, preferably, from 10 to 25% by weight based on the total amount of the medicinally active ingredient, cellulose ether and wax. The problems caused by the formulation with the wax in an amount outside the above mentioned range are similar to those caused by the use of a too small or too large amount of the cellulose ether.

In step (a) of the inventive method, the above described cellulose ether and wax are uniformly blended with the medicinally active ingredient each in a specified amount and the thus obtained uniform mixture is then shaped into tablet in step (b). The method for tableting is not particularly limitative and can be according to a conventional procedure.

The tablet prepared in step (b) is then subjected to a heat treatment and then cooling in step (c) of the inventive method. The temperature for the heat treatment should be in the vicinity of the melting point of the wax in the tablet or, in particular, within mp-20 °C to below the melting point thereof. By this heat treatment, the wax is brought into a semi-molten state to envelop the particles of the active ingredient. The heat treatment is performed for at least 3 minutes or, usually, for 5 to 30 minutes.

To describe the actual procedure of the preparation of the sustained-release medicated tablets according to the inventive method, the active ingredient, cellulose ether and wax as well as other optional additives are thoroughly blended by using a suitable blending machine such as V-mixers to give a uniform mixture which is then tableted into tablets according to a conventional method. The tablets are then subjected to a heat treatment under the above mentioned conditions by using a suitable oven such as air-circulation ovens, microwave ovens into the desired sustained-release tablets. When an air-circulation oven is used for the heat treatment, the tablets heated in the oven at the specified temperature for a specified length of time are kept standing as such for cooling down to room temperature.

The above mentioned optional additives can be any of those conventionally used in solid medicament forms including excipients such as starch, lactose, lubricants such as magnesium stearate, coloring agents, e.g., pigments, and so on depending on the particular application of the tablets.

It should be noted that the sequential order of the above described steps (b) and (c) can be reversed to give substantially the same results. Namely, the mixture of the components prepared in step (a) is first subjected to the heat treatment in the specified conditions and then shaped into tablets.

In the following, the method of the present invention is described in more detail by way of examples and comparative examples.

### Example 1.

Acetaminophen, HPMC (60SH-4000, a product by Shin-Etsu Chemical Co.) and hardened beef tallow melting at 60 °C were taken in a weight proportion of 70.2:21.3:8.5 and thoroughly blended for 20 minutes in a V-mixer to give a uniform mixture which was tableted into tablets of 8 mm diameter each weighing 235 mg and containing 165 mg of acetaminophen, 50 mg of HPMC and 20 mg of hardened beef tallow. The thus prepared tablets were divided into five groups, of which the first group, referred to as the Tablet I hereinbelow, was retained as such for the subsequent testing while the other four groups, referred to as the Tablets II, III, IV and V hereinbelow, were heated for 30 minutes in an air-circulation oven at a temperature of 40 °C, 50 °C, 60 °C and 70 °C, respectively, followed by cooling to room temperature prior to testing.

For comparison, another lot of tablets, referred to as the Tablet VI hereinbelow, was prepared from acetaminophen and the same HPMC as used above in a weight proportion of 70.2:29.8. The Tablet VI each weighing 235 mg had a diameter of 8 mm and contained 165.0 mg of acetaminophen and 70.0 mg of the HPMC. These tablets were used as such in the subsequent testing described below.

The Tablets I to VI were each subjected to the releasing test of the active ingredient according to the paddle method specified in Eleventh Japanese Pharmacopoeia using 900 ml of the First Solution of the pharmacopoeia having a pH of 1.2 at 100 rpm of the paddle revolution. The results of the test are shown in Table 1 giving the accumulated amounts in % of the released acetaminophen up to 12 hours.

To test the storage stability of the tablets, the Tablets I and III were kept standing at 40 °C for 7 days and the tablets after this aging test, referred to as the Tablets Ia and IIIa, respectively, hereinbelow, were subjected to the releasing test in the same manner as above to give the results also shown in Table 1.

**Table 1**

| Tablet No. | Releasing time, hours | | | | | | |
|---|---|---|---|---|---|---|---|
| | 0.5 | 1 | 2 | 4 | 6 | 8 | 12 |
| I | 12.9% | 22.3% | 37.6% | 52.4% | 73.3% | 98.3% | 100% |
| Ia | 21.1% | 39.6% | 55.1% | 71.1% | 100% | - | - |
| II | 3.1% | 9.2% | 18.3% | 33.5% | 48.9% | 62.1% | 78.1% |
| III | 2.8% | 6.3% | 14.7% | 28.6% | 43.6% | 58.0% | 75.9% |
| IIIa | 3.8% | 7.3% | 14.9% | 28.1% | 47.5% | 65.8% | 85.7% |
| IV | 2.7% | 5.1% | 11.8% | 26.8% | 41.3% | 52.8% | 73.1% |
| V | 11.8% | 21.7% | 35.4% | 50.8% | 71.1% | 96.2% | 100% |
| VI | 29.3% | 36.8% | 45.9% | 61.2% | 71.8% | 80.8% | 95.5% |

As is understood from the results shown in Table 1, the releasing velocity of acetaminophen from the tablet was well suppressed, especially, in the early stage up to 0.5 hour in the Tablets II, III and IV prepared according to the inventive method as compared with the Tablets I, V and VI prepared for comparative purpose. Further, comparison of the results obtained with the Tablets Ia and IIIa leads to a conclusion that the tablets prepared according to the inventive method have greatly improved storage stability.

The advantages obtained by the method of the present invention can be summarized as follows.
1. Reliable and satisfactory releasing characteristics of the active ingredient can be obtained with the sustained-release medicated tablets prepared according to the inventive method. This is in contrast with the conventional sustained-release tablets in which satisfactorily controlled concentration of the active ingredient in blood can be obtained only by increasing the weight or size of each tablet or by increasing the frequency of administration of small tablets, in particular, when a large dose of the active ingredient is required or the active ingredient is a compound having high solubility.
2. In contrast to the limitation in the sustained-release tablets as a mere combination of the gel-matrix type and the wax-matrix type that premature disintegration of the tablet cannot be avoided, the sustained-release tablet prepared according to the inventive method is free from this problem and not disintegrated at the early stage after administration as a consequence of the heat treatment carried out under specific conditions.
3. The heat treatment has an effect to cause aging and stabilization of the tablet so that the releasing characteristic of the tablet is not affected under an adverse condition of high temperatures or after a long period of storage.
4. As a consequence, the medicated tablets can be formulated with an increased amount of the medicinally active ingredient.

## Claims

1. A method for the preparation of a sustained-release medicated tablet which comprises the steps of:
(a) blending a medicinally active ingredient with a non-ionic water-soluble cellulose ether and a wax to give a mixture, the amounts of the cellulose ether and the wax being in the ranges from 10 to 40 % by weight and from 5 to 30 % by weight, respectively, based on the total amount of the active ingredient, cellulose ether and wax;
(b) tableting the mixture into a tablet; and
(c) heating the tablet at a temperature lower than the melting point of the wax but not lower by more than 20 °C than said melting point for a length of time of at least 3 minutes and then cooling.

2. The method for the preparation of a sustained-release medicated tablet as claimed in claim 1 in which the non-ionic water-soluble cellulose ether is selected from the group consisting of hydroxypropyl methyl cellulose, hydroxypropyl cellulose and methyl cellulose.

3. The method for the preparation of a sustained-release medicated tablet as claimed in claim 1 in which the wax is selected from the group consisting of hardened oils, paraffins, natural waxes, higher alcohols and higher fatty acids having a melting point in the range from 50 to 90 °C.

4. The method for the preparation of a sustained-release medicated tablet as claimed in claim 1 in which the amount of the nonionic water-soluble cellulose ether is in the range from 15 to 25% by weight based on the total amount of the active ingredient, cellulose ether and wax.

5. The method for the preparation of a sustained-release medicated tablet as claimed in claim 3 in which the wax has a melting point in the range from 55 to 70 °C.

6. The method for the preparation of a sustained-release medicated tablet as claimed in claim 3 in which the wax is a hardened oil.

7. The method for the preparation of a sustained-release medicated tablet as claimed in claim 1 in which the amount of the wax is in the range from 10 to 25% by weight based on the total amount of the active ingredient, cellulose ether and wax.

8. The method for the preparation of a sustained-release medicated tablet as claimed in claim 1 in which the length of time for heating in step (c) is in the range from 5 minutes to 30 minutes.

## Patentansprüche

1. Ein Verfahren zur Herstellung einer arzneistoffhaltigen Tablette mit verzögerter Freigabe, welches die folgenden Stufen umfasst:
(a) Mischen eines medizinisch aktiven Inhaltsstoffs mit einem nichtionischen wasserlöslichen Celluloseäther und einem Wachs, so, dass eine Mischung entsteht, wobei die Mengen des Celluloseäthers und des Wachses im Bereich von 10-40 Gew.-% resp. von 5-30 Gew.-%, basierend auf der Gesamtmenge an aktivem Inhaltsstoff, Celluloseäther und Wachs liegen;
(b) Tablettieren der Mischung zu Tabletten; und
(c) Erhitzen der Tablette bei einer Temperatur, die unterhalb des Schmelzpunkts des Wachses liegt, aber nicht um mehr als 20°C tiefer als der genannte Schmelzpunkt, während einer Zeitdauer von mindestens 3 Minuten und anschliessendes Kühlen.

2. Das Verfahren zur Herstellung einer arzneistoffhaltigen Tablette mit verzögerter Freigabe gemäss Anspruch 1, in welchem der nichtionische wasserlösliche Celluloseäther ausgewählt ist aus der Gruppe bestehend aus Hydroxypropylmethylcellulose, Hydroxypropylcellulose und Methylcellulose.

3. Das Verfahren zur Herstellung einer arzneistoffhaltigen Tablette mit verzögerter Freigabe gemäss Anspruch 1, in welchem das Wachs ausgewählt ist aus der Gruppe bestehend aus gehärteten Oelen, Paraffinen, natürlichen Wachsen, höheren Alkoholen und höheren Fettsäuren, welche einen Schmelzpunkt im Bereich von 50-90°C haben.

4. Das Verfahren zur Herstellung einer arzneistoffhaltigen Tablette mit verzögerter Freigabe gemäss Anspruch 1, in welchem die Menge des nichtionischen wasserlöslichen Celluloseäthers im Bereich von 15-25 Gew.-% basierend auf der Gesamtmenge an aktivem Inhaltsstoff, Celluloseäther und Wachs, liegt.

5. Das Verfahren zur Herstellung einer arzneistoffhaltigen Tablette mit verzögerter Freigabe gemäss Anspruch 3, in welchem das Wachs einen Schmelzpunkt im Bereich von 55-70°C hat.

6. Das Verfahren zur Herstellung einer arzneistoffhaltigen Tablette mit verzögerter Freigabe gemäss Anspruch 3, in welchem das Wachs ein gehärtetes Oel ist.

7. Das Verfahren zur Herstellung einer arzneistoffhaltigen Tablette mit verzögerter Freigabe gemäss Anspruch 1, in welchem die Menge des Wachses im Bereich von 10-25 Gew.-%, basierend auf der Gesamtmenge an aktivem Inhaltsstoff, Celluloseäther und Wachs, liegt.

8. Verfahren zur Herstellung einer arzneistoffhaltigen Tablette mit verzögerter Freigabe gemäss Anspruch 1, in welchem die Zeitdauer für die Erhitzungsstufe (c) im Bereich von 5 Minuten bis 30 Minuten liegt.

## Revendications

1. Procédé pour la préparation d'un comprimé médicamenteux à libération retardée qui comprend les étapes consistant à :
(a) mélanger un principe actif médicinalement avec un éther de cellulose hydro-soluble non ionique et une cire pour donner un mélange, les quantités de l'éther de cellulose et de la cire se situant dans les plages de 10 à 40 % en poids et de 5 à 30 % en poids respectivement, rapportés à la quantité totale du principe actif, éther de cellulose et cire ;
(b) former le mélange en comprimés ; et
(c) chauffer le comprimé à une température inférieure au point de fusion de la cire mais non inférieure de plus de 20°C à ce point de fusion pendant une durée d'au moins 3 minutes puis refroidissement.

2. Procédé pour la préparation d'un comprimé médicamenteux à libération retardée selon la revendication 1, dans lequel l'éther de cellulose hydrosoluble non ionique est choisi dans le groupe constitué par l'hydroxypropylméthylcellulose, l'hydroxypropylcellulose et la méthylcellulose.

3. Procédé pour la préparation d'un comprimé médicamenteux à libération retardée selon la revendication 1, dans lequel la cire est choisie dans le groupe constitué par des huiles durcies, des cires, des cires naturelles, des alcools supérieurs, et des acides gras supérieurs ayant un point de fusion dans la plage 50 à 90°C.

4. Procédé pour la préparation d'un comprimé médicamenteux à libération retardée selon la revendication 1, dans lequel la quantité de l'éther de cellulose hydrosoluble non ionique se situe dans la plage de 15 à 25 % en poids rapportés à la quantité totale du principe actif, éther de cellulose et cire.

5. Procédé pour la préparation d'un comprimé médicamenteux à libération retardée selon la revendication 3, dans lequel la cire a un point de fusion dans la plage de 55 à 70°C.

6. Procédé pour la préparation d'un comprimé médicamenteux à libération retardée selon la revendication 3, dans lequel la cire est une huile durcie.

7. Procédé pour la préparation d'un comprimé médicamenteux à libération retardée selon la revendication 1, dans lequel la quantité de cire se situe dans la plage de 10 à 25 % en poids rapporté à la quantité totale du principe actif, éther de cellulose et cire.

8. Procédé pour la préparation d'un comprimé médicamenteux à libération retardée selon la revendication 1, dans lequel la durée de chauffage à l'état (c) se situe dans la plage de 5 minutes à 30 minutes.
